Europäisches Patentamt

European Patent Office

Office européen des brevets

(11) Publication number: **0 327 765**
**A1**

## (12) EUROPEAN PATENT APPLICATION

(21) Application number: **88311871.3**

(22) Date of filing: **15.12.88**

(51) Int. Cl.⁴: **A61K 37/22 , A23L 1/30 ,**
**C11B 3/00**

(30) Priority: **16.12.87 IL 84840**

(43) Date of publication of application:
**16.08.89 Bulletin 89/33**

(84) Designated Contracting States:
**AT BE CH DE ES FR GB GR IT LI LU NL SE**

(71) Applicant: **INNOLAB N.V.**
**c/o Pierson, Heldring & Pierson (Curacao)**
**N.V. 6 John B. Gorsiraweg P.O. Box 3889**
**Curacao(AN)**

(72) Inventor: **Shenfeld, Avner**
**16 Hess Street**
**Rehovot(IL)**

(74) Representative: **Mercer, Christopher Paul et al**
**Carpmaels & Ransford 43, Bloomsbury**
**Square**
**London WC1A 2RA(GB)**

(54) **Dietary supplement and method for the treatment of menopause and manifestations of aging.**

(57) The present invention provides a dietary supplement containing a mixture of lipids, extracted from natural sources, which has a beneficial effect on a variety of disorders and syndromes in humans. The dietary supplement of the present invention is preferably formulated as a beverage, but may be formulated in granule, capsule or suppository form.

The dietary supplement of the present invention provides a combination of phospholipid and neutral lipid supplements to the diet. This dietary supplementation alleviates to a great extent the occurrence and severity of the hot flush syndrome in menopausal women. Furthermore, these supplements slow the deterioration of mental processes and to markedly improve memory, especially short-term memory, of individuals who have taken the supplement of the present invention.

EP 0 327 765 A1

# DIETARY SUPPLEMENT AND METHOD FOR THE TREATMENT OF MENOPAUSE AND MANIFESTATIONS OF AGING

Field of the Invention

This invention relates to compositions comprised of natural ingredients which exhibit a pronounced beneficial effect on certain conditions including reducing the intensity and frequency of menopausal hot flushes. Compositions of the present invention also have been shown to enhance the memory in humans and to reduce certain adverse effects on human mental processes that are associated with the aging process.

This invention also relates to methods for reducing the intensity and frequency of menopausal hot flushes, treating vaginal dryness and enhancing the memory and mental processes of humans, which have been diminished during the aging process, by administering the compositions of the present invention.

Background of the Invention

The aging process is known to deteriorate certain body functions; among these, the ability of women to produce ova for reproduction. Associated with the deterioration of ova production is estrogen withdrawal and vaginal dryness. The hot flush is a physiological response to estrogen withdrawal which often accompanies deteriorating reproductive function during menopause. Hot flushes are associated with a hormonal imbalance and result from tissue aging.

The hot flush is common to about 75% of menopausal women, and usually occurs in women between the ages of 45 and 55. About 30% of all women in this age group suffer from severe flushes for a certain period of time ranging from about 6 months to about 5 years. Five percent (5%) of all women continue to suffer from the syndrome for the remainder of their lives.

Estrogen treatment is effective in many cases, but there is controversy amongst physicians as to the duration and the side effects of such treatment. Presently, there is no alternative treatment for relieving hot flush syndrome in women other than estrogen treatment.

Other body functions, including those associated with mental processes and especially memory, also deteriorate with age. The loss of certain mental processes differs from one individual to another within the same age group. It is estimated that about 50% of people over the age of 65 suffer from a certain degree of memory loss, and about 15% of those people suffer from a severe degree of such mental dysfunction. It is a very distressing syndrome and little can be done to alleviate the deterioration.

It is an object of the present invention to provide novel dietary supplements which may be taken to alleviate the occurrence and severity of hot flushes in women and to improve the memory and mental processes of individuals.

It is an additional object of the present invention to provide a method for alleviating the occurrence and severity of hot flushes in women which are associated with hormonal imbalance and tissue aging.

It is a further object of the present invention to provide a method for improving memory, especially short-term memory and other mental dysfunction associated with the aging process.

Brief Description of the Invention

The present invention provides a dietary supplement composition containing a mixture of lipids, extracted from natural sources, which has a beneficial effect on a variety of disorders and syndromes in humans. The dietary supplement of the present invention is preferably formulated as a beverage, but may be formulated in granule, capsule or suppository form.

The dietary supplement composition of the present invention provides a combination of phospholipid and neutral lipid supplements to the diet. This dietary supplementation alleviates to a great extent the occurrence and severity of the hot flush syndrome. Furthermore, these supplements slow the deterioration of mental processes and markedly improve memory, especially short-term memory, of individuals who have taken the supplement of the present invention.

The lipid supplement of the present invention is a mixture of phospholipids and glycerides from animal and plant sources having a defined composition profile of fatty acids and phosphlipid head groups which are essential for proper effectiveness.

In relevant part, compositions of the present invention comprise:

a) a lipid mixture obtained from egg yolks, said lipid mixture containing a mixture of phospholipids comprising about 5% to about 40% by weight of the total weight of phospholipids and neutral lipids in said supplement;

b) a phospholipid mixture obtained from a source other than egg yolks in an amount equal to about 5% to about 40% by weight of the total weight of phospholipids and neutral lipids in said supplement wherein at least about 30% by weight of the total phospholipid content of said phospholipid mixture is comprised of negatively charged phospholipids;

c) a neutral lipid mixture obtained from a source other than egg yolks in an amount equal to about 20% to about 80% by weight of the total weight of phospholipids and neutral lipids in said supplement, and wherein said lipid mixture, said phospholipid mixture, and said neutral lipid mixture together comprise at least about 5% by weight of said supplement, said negatively charged phospholipids comprise at least about 10% by weight of said phospholipids and neutral lipids in said supplement and the weight ratio of neutral lipids to phospholipids in the supplement ranges from about 1:1 to about 2.5:1.

These dietary supplement compositions may be further comprised of antioxidants, and formulated with alcohol as beverages for ingestion. Certain additives, for example flavorings and/or coloring agents, may be added to make the supplement more palatable or to enhance the nutritional value of the supplement. Diluents and excipients may be added to the compositions of the present invention to produce a dietary supplement that may be used in capsule, granule or suppository form.

Compositions of the present invention are useful as dietary supplements for alleviating the occurrence and severity of hot flushes in menopausal women. In addition, these compositions are effective as dietary supplements for slowing, and in certain cases, reversing the deterioration of mental processes associated with the process of aging.

The present invention also relates to methods for alleviating the occurrence and severity of hot flushes in women which are associated with hormonal imbalance during and after menopause and tissue aging.

The present invention further relates to a method for improving memory, especially short-term memory and other mental dysfunction associated with the aging process.

Detailed Description of the Invention

Dietary supplement compositions of the present invention comprise a lipid mixture obtained from egg yolks, a phospholipid mixture obtained from a source other than egg yolk, preferably having a high percentage of negatively charged phospholipids, and a neutral lipid mixture obtained from a source other than egg yolks, preferably containing a high percentage of neutral lipids. Preferred embodiments additionally comprise an effective amount of a physiologically acceptable antioxidant and/or at least about 0.5% of ethyl alcohol.

The lipid mixture is a mixture of phospholipids and neutral lipids obtained from egg yolk by extraction. The lipid mixture may be obtained by extracting egg yolks with any number of non-polar and/or polar solvents including chloroform, methylene chloride, acetone, chloroform-methanol 1:1 v/v, tetrahydrofuran, diethyl ether, diethyl ketone, acetonitrile, methanol and ethanol, among others, followed by precipitation of a certain fraction of the extracted lipid, preferably with acetone. Such a procedure, described in U.S. Patent No. 4,474,773, which is hereby incorporated by reference, produces a lipid mixture of phospholipids and neutral lipids wherein the percentage of neutral lipids is relatively high compared to the phospholipid component (weight ratio of neutral lipids to phospholipids generally ranges from about 1:1 to about 7:3). AL-721™ (Ethigen Corporation, U.S.A.), described in U.S. Patent No. 4,474,773, is an example of a commercially available product produced using such a procedure.

Alternatively, and preferably, the lipid mixture obtained from egg yolk contains a high percentage of phospholipid compared to neutral lipid. In order to produce such a lipid mixture, it is preferable to extract the egg yolks with a polar solvent, preferably boiling ethyl alcohol, for a period of about one hour. It is preferred that 95% ethanol be used, but an ethanol-water mixture containing at least about 70% ethanol may also be used to effect the extraction. Other polar solvents may be used, but preferred solvents include those which are pharmaceutically compatible and are non-toxic in small amounts. The extraction of egg yolks with a polar solvent produces a mixture of phospholipids and neutral lipids in which phospholipids, more polar than the neutral lipids, predominate. Preferably, the weight ratio of phospholipids to neutral lipids in the lipid mixture obtained from egg yolk is about 1:2.

3

The lipid mixture obtained from egg yolk comprises about 5% to about 40%, and preferably, about 10% to about 30% by weight of the total weight of phospholipids and neutral lipid sin the compositions. The amount of lipid mixture obtained from the egg yolks generally equals about 5% to about 15% by weight of the total weight of crude egg yolk.

Compositions of the present invention also comprise a phospholipid mixture obtained from a source other than egg yolks containing a mixture of phospholipids wherein at least about 30% and preferably at least about 50% of the phospholipids are negatively charged phospholipids. Any number of non-egg yolk sources of phospholipids are available including, for example, vegetable or soya lecithin (commercial granulated soya lecithin) and brain phospholipids. The phospholipid source is selected such that at least 30% by weight of the phospholipids are negatively charged phospholipids. Negatively charged phospholipids are those phospholipids that have a net negative charge and include phosphatidic acid, phosphatidylserine, phosphatidylinositol and phosphatidylglycerol, among other phospholipids. Of those phospholipids that are not negatively charged, phosphatidylcholine and phosphatidylethanolamine predominate. Preferably, the weight ratio of phosphatidylcholine to phosphatidylethanolamine in the phospholipid mixture is about 1:1, but may vary over a wide range. The amount of phospholipid mixture included in the compositions of the present invention varies, but generally ranges between about 5% and about 40%, preferably 20% to 40%, of the total weight of phospholipids and neutral lipids in the composition. The amount of the phospholipid mixture included in the compositions of the present invention is such that the total amount of negatively charged phospholipids is at least about 10% by weight of the total weight of phospholipids and neutral lipids.

The inclusion of relatively large amounts of negatively charged phospholipids is viewed as important to the activity of the compositions. By including large amounts of negatively charged phospholipids above about 10% of the total weight of phospholipids and neutral lipids, compositions of the present invention may act to provide a source of negatively charged phospholipids which disappear during the aging process, especially in the brain. The inclusion of high percentages of negatively charged phospholipids in supplement compositions of the present invention acts to reverse this disappearance.

Compositions of the present invention also comprise a neutral lipid mixture obtained from a non-egg yolk source. The amount of neutral lipid included in compositions of the .present invention ranges from about 20% to about 80% of the total weight of phospholipids and neutral lipids in the composition. Preferably, compositions of the present invention comprise about 30 to about 50% neutral lipids. Neutral lipids include mono-glycerides, di-glycerides and tri-glycerides as well as cholesterol, carotene and other hydrophobic pigments. Preferable sources of neutral lipids include butter, fish oil and cocoanut oil. While a number of mixtures of neutral lipids may be used in practicing the present invention, it is preferred that a source of neutral lipids which comprises predominantly (greater than 50% by weight) short-chain neutral lipids is used. Short-chain neutral lipids are mono, di, and tri-glycerides having side chains of less than 12 carbon units and include, for example glycerol tributyrate (tributyrene). Short-chain neutral lipids are preferred because they function as potent precursors for phospholipid and neutral lipid synthesis.

It is preferred that the neutral lipid mixture comprises about 30% to about 50% by weight of the total weight of phospholipids and neutral lipids. The amount of neutral lipid incorporated into compositions of the present invention is chosen so that the weight ratio of neutral lipid to phospholipid ranges between about 1:1 to about 2.5:1. It is especially preferred that the weight ratio of neutral lipids to phospholipids in compositions of the present invention is about 2:1.

Compositions of the present invention may include a physiologically compatible antioxidant, for example, alpha-tocopherol, tocopherylacetate and ascorbic acid, among others. It is preferred that the antioxidant comprises between about 0.2% to about 2.0% by weight of the phospholipids and neutral lipids, and, most preferably, the antioxidant comprises about 0.5% by weight of the phospholipids and neutral lipids.

Ethyl alcohol is preferably included in liquid dietary supplements formulated with compositions of the present invention. In the liquid supplement, the alcohol preferably comprises at least about 0.5% v/w of the liquid supplement. The alcohol is included in the supplement to aid the emulsification of the lipid mixtures (phospholipids and neutral lipids), to provide preservative and anti-bacterial activity and to function as a fluidizer in conjunction with the lipid mixtures. The ethyl alcohol appears to act synergistically with lipid mixture compositions of the present invention.

Compositions of the present invention are preferably provided as a liquid dietary supplement or alternatively, these compositions may be formulated as granules, capsules or suppositories. The liquid supplement may include a number of suitable carriers and additives including water, glycols, oils, alcohols, flavoring agents, preservatives, coloring agents and the like. In capsule, granule or suppository form, the compositions of the present invention are formulated in admixture with a pharmaceutically acceptable

4

carrier.

To prepare the compositions of the present invention in capsule, granule or suppository form, one or more compositions of the present invention may be intimately admixed with a pharmaceutically acceptable carrier according to conventional formulation techniques. For solid oral preparations such as capsules and granules, suitable carriers and additives such as starches, sugars, diluents, granulating agents, lubricants, binders, disintegrating agents and the like may be included.

Compositions of the present invention are ingested in liquid form or are taken in capsule, granule or suppository form once or twice a day. Dosages generally should range from about 10 to 50 g. per day depending on the composition ingested. In the most preferred embodiment of the present invention, the daily dose should be about 24 g. per day. The compositions are preferably ingested once daily in the morning or the evening on an empty stomach or with non-fat foods at least two hours before eating.

The present invention also relates to methods for alleviating the occurrence and severity of hot flushes associated with estrogen withdrawal in menopausal women and for slowing the deterioration of mental processes and improving memory, especially short-term memory.

In the methods of the present invention, the lipid mixture compositions of the present invention may be used to alleviate the occurrence and severity of hot flushes in menopausal women. These same compositions may be used to improve short-term memory and other mental dysfunction caused by the process of aging. In a further embodiment of the present invention, the commercially available egg lecithin product Al-721TM is used to alleviate hot flushes in women and to restore the short-term memory to individuals with mental dysfunction produced by the aging process.

In the method of the present invention compositions of the present invention, as well as AL-721TM, are provided as a dietary supplement in doses of about 24 grams of total lipid for periods of about two weeks to individuals exhibiting hot flushes or loss of short-term memory. The method of the present invention results in alleviation of the debilitation associated with these conditions.

AL-721TM egg lecithin is an lipid mixture extract from egg yolks comprising about 70% of a neutral lipid fraction, and about 30% of phospholipids (phosphatidylcholine and phosphatidylethanolamine in a weight ratio of about 2:1).

The present invention may be further understood by way of the following examples. It is to be understood by those skilled in the art that the following examples are illustrative of practicing the present invention, but in no way should they be considered limiting.

Example 1

A liquid dietary preparation was prepared as follows: Twenty egg-yolks (360 g.) were introduced into 6 liters of ethyl alcohol (95%) and boiled for one hour. The heated mixture was filtered to remove undesired residues. To the resulting liquid was added 100 grams of soya lecithin (commercial granulated soya lecithin, about 70% phospholipids), 100 grams of butter and 2 grams of vitamin E as an anti-oxidant. The mixture was thoroughly homogenized and the alcohol content was evaporated to a residual alcohol content of from about 10 to about 20%. The recovered alcohol was recycled and used with additional batches. To the residual material was added a quantity of water up to a final volume of 3 liters. Additives including flavoring such as vanilla or cocoa, coloring and additional additives may be used to increase the palatability of the formulation. Formulations having a low alcohol content have a pleasant taste.

The final product is an aqueous emulsion which includes about 12% (w/v) of lipids as the active ingredient, 5-8% ethyl alcohol and a mixture of antioxidants.

Example 2

The weight ratio of the egg-yolk lipid mixture, the phospholipid mixture and the neutral lipid mixture may be varied according to the scope of the present invention to produce products containing the following weight ratios of the lipid components:

| Component | Amount Percent by Weight of Total Lipid Mixture |
|---|---|
| a) lipid mixture from egg yolk | 5 to 40% |
| b) phospholipid mixture from vegetable source containing at least about 30% by weight of negatively-charged phospholipids | 5 to 40% |
| c) neutral lipids | 20 to 80% |
| d) antioxidant | 0.2 to 2.0% |

These compositions may be formulated as a liquid dietary supplement containing preferably at least about 0.5% v/w of the liquid supplement and, most preferably, about 5% to about 8%, along with flavorings, colorings and the like, or alternatively, these compositions may be formulated using standard pharmaceutical formulation techniques to produce a dietary supplement in granule, capsule or suppository form.

Example 3

Effect of Lipid Mixture Compositions On Hot Flushes of Menopausal Women

The composition of Example 1 was tested in a group of 12 women who were measured for the number and intensity of daily hot flushes. Each of the women was subjected to 4 test periods with an interval between each of these periods. For period A, the number and intensity of hot flushes were measured in the group of 12 women who had not received the composition of Example 1. For period B, the number and intensity of hot flushes were measured in the same group of 12 women after ingesting a cocktail mixture comprising 12 g. of lipid mixture (100 ml. of Example 1) daily for a period of 2 weeks. For period C, the number and intensity of hot flushes in the group of 12 women were measured after ingesting a cocktail mixture comprising 24 g. of lipid mixture. For period D, the number and intensity of hot flushes were measured 3 to 10 days after the lipid mixture compositions were withdrawn.

Results of the test (Table 1) demonstrate a pronounced decrease in the severity of the flushes, as well as in their total number. After termination of the administration of the liquid dietary supplement of Example 1 (period D), a partial relapse occurred.

Table 1

| Daily Number and Intensity of Hot Flushes in 12 Women | | | | | |
|---|---|---|---|---|---|
| Intensity/Period | Mild | Moderate | Severe | Total | Weighted Mean ±S.D. |
| A | 0.75 | 5.0 | 10.5 | 6.3 | 42.3 ±15.5 |
| B | 3.5 | 2.5 | 2.5 | 8.5 | 16.0 ± 9.0 |
| C | 3.5 | 1.5 | 2.3 | 7.3 | 13.4 ±10.0 |
| D | 2.5 | 4.8 | 4.2 | 11.5 | 24.7 ±11.0 |
| Period of Test: | | | | | |
| A- control | | | | | |
| B- 12 g. Cocktail Mixture (Example 1) daily for 2 weeks | | | | | |
| C- 24 g. Cocktail mixture (Example 1) daily for 2 weeks | | | | | |
| D- 3 to 10 days after withdrawal of medication | | | | | |

Each of the women passed 4 sequential periods of tests as indicated above. Hot flushes were scored by the participating subjects using three parameters: temperature, sweat and tremors. The results show a marked reduction in the severity of the flushes, as well as in their total number. Shortly after withdrawal of medication, a partial relapse was observed.

The serum lipid components including cholesterol, triglycerides, high density lipoproteins (HDL) and low density lipoprotein (LDL) of the 12 women were determined during the trial periods. These are presented in Table 2, below. The results demonstrate that no substantial change of serum lipids took place.

The levels of the hormones FSA and beta-estradiol in the plasma were also determined. The results, summarized in Table 3, indicate that there was no appreciable change in hormone levels as a result of the ingestion of these lipids.

Table 2

| Serum Components (mg/ml) (Mean + S.D.) During The Study Period (1st Trial) | | | | |
|---|---|---|---|---|
| Serum Components | PERIOD | | | |
| | A | B | C | D |
| Total Cholesterol | 252 | 256 | 258 | 268.5 |
| Triglycerides | 165 | 178 | 227 | 207.7 |
| Total Cholesterol | 4.8 | 4.9 | 5.0 | 4.5 |
| HDL | 60 | 57 | 55 | 62 |
| LDL | 3.0 | 2.9 ± 0.3 | 3.0 ± 0.5 | 2.7 |
| Period of Test: | | | | |
| A- control | | | | |
| B- 12 g. Cocktail Mixture (Example 1) daily for 2 weeks | | | | |
| C- 24 g. Cocktail mixture (Example 1) daily for 2 weeks | | | | |
| D- 3 to 10 days after withdrawal of medication | | | | |

Table 3

| Mean Values of FSH (mU/ml) and 17 beta-estradiol (pg/ml) During Study Period (First Trial) | | |
|---|---|---|
| Period | FSH | 17 beta-estradiol |
| A | 99 | 33 |
| B | 106 | 32 |
| C | 98 | 35 |
| D | 100 | 31 |
| Period of Test: | | |
| A- control | | |
| B- 12 g. Cocktail Mixture (Example 1) daily for 2 weeks | | |
| C- 24 g. Cocktail mixture (Example 1) daily for 2 weeks | | |
| D- 3 to 10 days after withdrawal of medication | | |

## Example 4

Effect of Lipid Mixture Composition AL-721™ On Hot Flushes of Menopausal Women

The lipid mixture composition AL-721™ was tested in a group of 10 women who were measured for the number and intensity of daily hot flushes. Each of the women was subjected to 3 test periods with an interval between each of these periods. For period A, the number and intensity of hot flushes were measured in women who had not received AL-721™. For period B, the number and intensity of hot flushes were measured in the same group of 10 women after ingesting 15 g. of AL-721™ daily for a period of 2 weeks. For period C, the number and intensity of hot flushes were measured 3 days after AL-721™ was withdrawn.

Results of the test demonstrate (Table 4) a significant decrease in the severity of the flushes, as well as in their total number. After termination of the administration of AL-721™ (period C), a partial relapse of hot flushes occurred.

Table 4

| Average Daily Number and Intensity of Hot Flushes in 10 Women Receiving AL-721™ | | | | |
|---|---|---|---|---|
| Period | INTENSITY | | | |
| | Mild | Moderate | Severe | Total |
| A | 1.2 | 4.6 | 8.6 | 14.4 |
| B | 2.2 | 3.6 | 1.6 | 7.4 |
| C | 3.5 | 3.2 | 3.8 | 10.5 |
| Period of Test: | | | | |
| A- before treatment | | | | |
| B- 15 g. of AL-721™ daily for 2 weeks | | | | |
| C- 3 days after withdrawal of AL-721™ | | | | |

This trial was carried out several months after trial number 1 (Example 3). Each of the women passed through three sequential periods of test as indicated. The results are similar to those presented in Table 1 and verify the use of AL-721™ for use as an agent to reduce hot flushes in menopausal women.

## Example 5

Effect of Example 1 Composition on Short Term Memory

Studies have shown that after ingestion of 24 g. of the composition of Example 1 for a period of a few weeks, improvement of memory took place and the general well-being of the persons treated improved. Small-scale trials on individuals suffering from loss of short-term memory and other mental dysfunction indicate the pronounced efficacy of the preparations of the invention in this respect. The trials were carried out on an individual basis and results were based on the individuals reporting subjective improvements during the studies.

It is to be understood by those skilled in the art that the foregoing description and examples are illustrative of practicing the present invention, but are in no way limiting. Variations of the detail presented herein may be made without departing from the spirit and scope of the present invention.

**Claims**

1. A dietary supplement for ingestion by humans comprising:

a) a lipid mixture obtained from egg yolks by extracting said egg yolks with an organic solvent, said lipid mixture containing a mixture of phospholipids comprising about 5% to about 40% by weight of the total weight of phospholipids and neutral lipids in said supplement;

b) a phospholipid mixture obtained from a source other than egg yolks in an amount equal to about 5% to about 40% by weight of the total weight of phospholipids and neutral lipids in said supplement wherein at least about 30% by weight of the total phospholipid content of said phospholipid mixture is comprised of negatively charged phospholipids; and

c) a neutral lipid mixture obtained from a source other than egg yolks in an amount equal to about 20% to about 80% by weight of the total weight of phospholipids and neutral lipids in said supplement, and wherein said lipid mixture, said phospholipid mixture, and said neutral lipid mixture together comprise at least about 5% by weight of said supplement, said negatively charged phospholipids comprise at least about 10% by weight of said phospholipids and neutral lipids of said supplement and the weight ratio of neutral lipids to phospholipids in said supplement ranges from about 1:1 to about 2.5:1.

2. The dietary supplement according to claim 1 further comprising at least about 0.5% (v/w) of the total weight of the supplement of ethyl alcohol.

3. The supplement according to claim 1 or 2 further comprising an effective amount of a physiologically acceptable antioxidant.

4. The supplement according to any of claims 1 through 3 wherein said antioxidant comprises about 0.2 to about 2.0% by weight.

5. The supplement according to any of claims 1 through 4 wherein said lipid mixture is obtained by the extraction of egg yolks with boiling 95% ethyl alcohol.

6. The supplement according to any of claims 1 through 5 wherein said phospholipid mixture is soya lecithin.

7. The supplement according to any of claims 1 through 6 wherein said neutral lipid mixture is obtained from animal butter.

8. The supplement according to any of claims 1 through 7 wherein said neutral lipid mixure is predominantly comprised of short-chain neutral lipids.

9. A dietary supplement for ingestion by humans comprising:

a) a lipid mixture obtained from egg yolks by extracting said egg yolks with boiling ethanol for a period of about one hour, said lipid mixture containing a mixture of phospholipids comprising about 5% to about 40% by weight of the total weight of phospholipids and neutral lipids in said supplement;

b) a phospholipid mixture obtained from a source other than egg yolk in an amount equal to about 5% to about 40% by weight of the total weight of phospholipids and neutral lipids in said supplement wherein at least about 30% by weight of the total phospholipid content of said phospholipid mixture is comprised of negatively charged phospholipids; and

c) a neutral lipid mixture obtained from a source other than egg yolk in an amount equal to about 20% to about 80% by weight of the total weight of phospholipids and neutral lipids in said supplement, and wherein said lipid mixture, said phospholipid mixture, and said neutral lipid mixture together comprise at least about 5% by weight of said supplement, said negatively charged phospholipids comprise at least about 10% by weight of said phospholipids and said neutral lipids.

10. The supplement according to claim 9 wherein the weight ratio of neutral lipids to phospholipids in said supplement ranges from about 1:1 to about 2.5:1.

11. The supplement according to claims 9 or 10 wherein said phospholipid mixture is obtained from soya lecithin and said neutral lipid mixture is obtained from animal butter.

12. A dietary supplement for use in treating hot flushes in menopausal women comprising comprising:

a) a lipid mixture obtained from egg yolks by extracting said egg yolks with an organic solvent, said lipid mixture containing a mixture of phospholipids comprising about 5% to about 40% by weight of the total weight of phospholipids and neutral lipids in said supplement;

9

b) a phospholipid mixture obtained from a source other than egg yolks in an amount equal to about 5% to about 40% by weight of the total weight of phospholipids and neutral lipids in said supplement wherein at least about 30% by weight of the total phospholipid content of said phospholipid mixture is comprised of negatively charged phospholipids; and

c) a neutral lipid mixture obtained from a source other than egg yolks in an amount equal to about 20% to about 80% by weight of the total weight of phospholipids and neutral lipids in said supplement, and wherein said lipid mixture, said phospholipid mixture, and said neutral lipid mixture together comprise at least about 5% by weight of said supplement, said negatively charged phospholipids comprise at least about 10% by weight of said phospholipids and neutral lipids of said supplement and the weight ratio of neutral lipids to phospholipids in said supplement ranges from about 1:1 to about 2.5:1.

13. The supplement according to claim 12 wherein said lipid mixture comprises about 20% to about 40% by weight, said phospholipid mixture comprises about 20% to about 40%, and said neutral lipid mixture comprises about 30% to about 50% by weight of said phospholipids and neutral lipids.

14. The supplement according to claims 12 or 13 wherein said supplement further comprises a pharmaceutically compatible antioxidant.

15. The supplement according to any of claims 12 through 14 wherein said supplement further comprises at least about 0.5% v/w of the total weight of the supplement of ethyl alcohol.

16. The supplement according to any of claims 12 through 15 wherein said lipid mixture is obtained by extracting egg yolks with boiling ethanol for a period of one hour.

17. The supplement according to any of claims 12 through 16 wherein said phospholipid mixture is soya lecithin.

18. The supplement according to any of claims 12 through 17 wherein said neutral lipid mixture is obtained from animal butter.

19. The supplement according to any of claims 12 through 18 wherein said neutral lipid mixture is predominantly comprised of short-chain neutral lipids.

20. A dietary supplement for use in treating mental dysfunction including loss of short-term memory caused by the aging process in humans comprising:

a) a lipid mixture obtained from egg yolks by extracting said egg yolks with an organic solvent, said lipid mixture containing a mixture of phospholipids comprising about 5% to about 40% by weight of the total weight of phospholipids and neutral lipids in said supplement;

b) a phospholipid mixture obtained from a source other than egg yolks in an amount equal to about 5% to about 40% by weight of the total weight of phospholipids and neutral lipids in said supplement wherein at least about 30% by weight of the total phospholipid content of said phospholipid mixture is comprised of negatively charged phospholipids; and

c) a neutral lipid mixture obtained from a source other than egg yolks in an amount equal to about 20% to about 80% by weight of the total weight of phospholipids and neutral lipids in said supplement, and wherein said lipid mixture, said phospholipid mixture, and said neutral lipid mixture together comprise at least about 5% by weight of said supplement, said negatively charged phospholipids comprise at least about 10% by weight of said phospholipids and neutral lipids of said supplement and the weight ratio of neutral lipids to phospholipids in said supplement ranges from about 1:1 to about 2.5:1.

21. The supplement according to claim 20 wherein said lipid mixture comprises about 20% to about 40% by weight, said phospholipid mixture comprises about 20% to about 40%, and said neutral lipid mixture comprises about 30% to about 50% by weight of said phospholipids and neutral lipids.

22. The supplement according to claims 20 or 21 wherein said supplement further comprises a pharmaceutically compatible antioxidant.

23. The supplement according to any of claims 20 through 22 wherein said supplement further comprises at least about 0.5% v/w of the total weight of the supplement of ethyl alcohol.

24. The supplement according to any of claims 20 through 23 wherein said lipid mixture is obtained by extracting egg yolks with boiling ethanol for a period of one hour.

25. The supplement according to any of claims 20 through 24 wherein said supplement is administered to said human for a period of at least two weeks.

26. A method of treating hot flushes in menopausal women comprising administering to a woman experiencing hot flushes at least about 15 g. daily of AL-721TM for a period of at least about two weeks.

27. A method of treating mental dysfunction including loss of short-term memory caused by the aging process in humans comprising administering to an individual experiencing a loss of memory at least about 15 g. daily of AL-721TM for a period of at least about two weeks.

28. A method of making a dietary supplement for ingestion by humans comprising:

a) extracting egg yolks with an organic solvent to produce a lipid mixture containing a mixture of phospholipids and neutral lipids comprising about 5% to about 40% by weight of the total weight of phospholipids and neutral lipids in said supplement;

b) adding to said egg yolk

i) a phospholipid mixture obtained from a source other than egg yolks in an amount equal to about 5% to about 40% by weight of the total weight of phospholipids and neutral lipids in said supplement wherein at least about 30% by weight of the total phospholipid content of said phospholipid mixture is comprised of negatively charged phospholipids and

ii) a neutral lipid mixture obtained from a source other than egg yolks in an amount equal to about 20% to about 80% by weight of the total weight of phospholipids and neutral lipids in said supplement, and wherein said lipid mixture, said phospholipid mixture, and said neutral lipid mixture together comprise at least about 5% by weight of said supplement, said negatively charged phospholipids comprise at least about 10% by weight of said phospholipids and neutral lipids of said supplement and the weight ratio of neutral lipids to phospholipids in said supplement ranges from about 1:1 to about 2.5:1; and

c) homogenizing said lipid mixture, said phospholipid mixture and said neutral lipid mixture.

29. The method according to claim 28 comprising the additional step of adding a pharmaceutically compatible antioxidant to said mixture from claim 28.

30. The method according to claims 28 or 29 comprising the additional step of adding ethyl alcohol to said mixture from claim 28.

31. A method of making a dietary supplement for ingestion by humans comprising:

a) extracting egg yolks with boiling ethyl alcohol for a period of about one hour to produce a lipid mixture containing a mixture of phospholipids and neutral lipids comprising about 5% to about 40% by weight of the total weight of phospholipids and neutral lipids in said supplement;

b) filtering said mixture to remove undesired residues;

c) adding to said egg yolk mixture

i) a phospholipid mixture obtained from a source other than egg yolks in an amount equal to about 5% to about 40% by weight of the total weight of phospholipids and neutral lipids in said supplement wherein at least about 30% by weight of the total phospholipid content of said phospholipid mixture is comprised of negatively charged phospholipids and

ii) a neutral lipid mixture obtained from a source other than egg yolks in an amount equal to about 20% to about 80% by weight of the total weight of phospholipids and neutral lipids in said supplement, and wherein said lipid mixture, said phospholipid mixture, and said neutral lipid mixture together comprise at least about 5% by weight of said supplement, said negatively charged phospholipids comprise at least about 10% by weight of said phospholipids and neutral lipids of said supplement and the weight ratio of neutral lipids to phospholipids in said supplement ranges from about 1:1 to about 2.5:1;

c) homogenizing said lipid mixture, said phospholipid mixture and said neutral lipid mixture; and

d) evaporating said ethyl alcohol to about 10% to about 20% of said ethyl alcohol used for extracting said egg yolks.

32. The method according to claim 31 comprising the additional step of adding a pharmaceutically compatible antioxidant to said mixture from claim 31.

European Patent Office

**PARTIAL EUROPEAN SEARCH REPORT**
which under Rule 45 of the European Patent Convention
shall be considered, for the purposes of subsequent
proceedings, as the European search report

Application number

EP 88 31 1871

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 074 251 (YEDA RESEARCH AND DEVELOPMENT) <br> * Claims 1-12,14,16; page 9, lines 20-34 * <br><br> & US-A- 4 474 773 (cat. D ) <br> -- | 1-3,4, 6,9,12, 17,20, 21,22, 28,31 | A 61 K 37/22 <br> A 23 L 1/30 <br> A 23 J 7/03 <br> C 11 B 3/00 |
| A | EP-A-0 148 303 (A. VON MLETZKO) <br> * Claims 1-9; page 5, lines 13-30; page 2, lines 13-26 * <br><br><br> -- | 1,6,8, 9,12, 13,17, 19,20, 21,28, 31 | |
| A | EP-A-0 185 235 (A. NATTERMAN & CIE) <br> * Claims 1-5 * <br><br> -- | 1,9,12, 20,28 | TECHNICAL FIELDS SEARCHED (Int. Cl.4) |
| A | EP-A-0 141 442 (UNILEVER) <br> * Claims 1-20 * | 1,9,12, 20,28 | A 61 K <br> A 23 L <br> A 23 J <br> C 11 B |

**INCOMPLETE SEARCH** -------

The Search Division considers that the present European patent application does not comply with
the provisions of the European Patent Convention to such an extent that it is not possible to carry
out a meaningful search into the state of the art on the basis of some of the claims.
Claims searched completely: 1-24,28-31
Claims searched incompletely:
Claims not searched: 25,26,27
Reason for the limitation of the search:

Method for treatment of the human
or animal body by surgery or therapy
(See art. 52(4) of the European
Patent Convention)

| Place of search | Date of completion of the search | Examiner |
|---|---|---|
| The Hague | 11-04-1989 | VAN MOER |

EPO Form 1505.1.03.82